# EUROPEAN PATENT SPECIFICATION

(11) **EP 3 573 577 B1**
(45) Date of publication and mention of the grant of the patent: **19.10.2022**
(21) Application number: 18704698.2
(22) Date of filing: 25.01.2018
(51) Int. Cl.: A61F 2/07, A61F 2/06, A61M 25/10

(54) **DEVICE FOR TREATMENT AND PREVENTION OF FLUID OVERLOAD IN PATIENTS WITH HEART FAILURE**
VORRICHTUNG ZUR BEHANDLUNG UND PRÄVENTION VON FLÜSSIGKEITSÜBERSCHUSS IN PATIENTEN MIT HERZINSUFFIZIENZ
DISPOSITIF DE TRAITEMENT ET DE PRÉVENTION DE LA SURCHARGE DE FLUIDE CHEZ DES PATIENTS PRÉSENTANT UNE INSUFFISANCE CARDIAQUE

(30) Priority: 25.01.2017 US 201762450434 P
(43) Date of publication of application: 04.12.2019
(73) Proprietor: W. L. Gore & Associates, Inc., Newark, DE 19711 (US)
(72) Inventor: GOODMAN, Paul, D., Newark Delaware 19711 (US); DUGDALE, Joel, W., Newark Delaware 19711 (US); TROKANSKI, Mark, E., Newark Delaware 19711 (US); VONESH, Michael, J., Newark Delaware 19711 (US); BRINKMANN, John, M., Newark Delaware 19711 (US)
(74) Representative: HGF
(86) International application number: PCT/US2018/015304
(87) International publication number: WO 2018/140637

(56) References cited:
- WO-A2-2007/127477
- US-A1- 2012 130 478
- US-A1- 2014 039 537

## Description

### TECHNICAL FIELD

The present disclosure relates to systems, medical devices, and methods for treating heart failure and/or other cardiovascular diseases. More specifically, the disclosure relates to removing buildup of excess fluid that increases pressure on a patient's heart.

### BACKGROUND

Patients experiencing heart failure may have a buildup of excess fluid in the body. The excess fluid buildup may increase fluid accumulation in the interstitial space and add stress on an already failing heart. Excess fluid (or hypervolemia) is the leading cause of hospitalization for heart failure patients (approximately 1,000,000 per year in the United States).

Treatment of the excess fluid buildup may be treated pharmaceutically by diuretics (or other pharmaceutical agents). However, a patient may experience drug resistance, inaccurate dosing, or other issues such as failure to comply with medicine directives. Non-pharmaceutical options, such as implantable device solutions that provide an alternative to or augment pharmaceutical efficacy by influencing renal function, may be beneficial to avoid these and other issues in treatment of buildup of excess fluid in the body. Similarly, chronic high blood pressure (hypertension) can also be managed pharmaceutically by antihypertensive medications (or other pharmaceutical agents). In addition, other disease states may result in hypotension, reduced cardiac output, and poor renal function. Insofar as the kidneys play a central role in regulating systemic blood pressure, non-pharmaceutical options, such as implantable device solutions that provide an alternative to or augment pharmaceutical efficacy by influencing renal function, may provide an alternative means of managing hypertension and other disease states.

(WO 2007/127477) is directed to Intravascular devices are delivered to the aorta percutaneously via the femoral artery. The devices are anchored within the vasculature in the region of the renal artery ostia. These embodiments function to increase the flow of blood from the aorta to the renal arteries, thus delivering a higher relative percentage of the blood flowing through the aorta to the kidneys. The elevation (WO 2007/127477) is directed to Intravascular devices are delivered to the aorta percutaneously via the femoral artery. The devices are anchored within the vasculature in the region of the renal artery ostia. These embodiments function to increase the flow of blood from the aorta to the renal arteries, thus delivering a higher relative percentage of the blood flowing through the aorta to the kidneys. The elevation in blood flow to the kidneys improves the natural removal of excess fluids from the body. US 2014/039537 is directed to devices for the control of flow rate and/or pressure within a vessel of a mammalian patient, and methods of treating an afflicted vessel and/or a vessel associated with an afflicted tissue using the devices. US 2012/130478 is directed to a stent-graft comprising a first portion that is configured to engage a vessel wall, a second portion that is configured not to engage the vessel wall, and a perfusion window that is configured to permit blood flow. The stent-graft may further comprise a transition portion between the first portion and the second portion, and the perfusion window may be formed in the first portion, the second portion, and/or the transition portion.

### SUMMARY

Various aspects of the present disclosure and not covered by the invention are directed toward methods that include arranging an implantable flow restriction device within the aorta of a patient. The methods may also include adjusting the implantable flow restriction device to increase blood flow into at least one branch vessel of the aorta while maintaining a substantially unrestricted blood flow within the aorta proximal to the branch vessel.

Aspects of the present disclosure are also directed toward apparatuses that include an implantable flow restriction device configured to implant with an aorta of a patient. The implantable flow restriction device may also be configured to alter blood flow into at least one branch vessel of the aorta while maintaining a substantially unrestricted blood flow within the aorta proximal to the branch vessel.

Various aspects of the present disclosure not covered by the invention are also directed toward methods for augmenting perfusion of a branch vessel originating from the aorta. The methods may include arranging an implantable flow restriction device with an aorta of a patient and adjusting the implantable flow restriction device to alter pressure within the aorta to alter blood flow into the branch vessel of the aorta while maintaining a substantially unrestricted blood flow within the aorta proximal to the branch vessel.

According to the invention, there is provided an apparatus including an implantable flow restriction device including a first portion, a second portion and a channel therebetween configured to: implant within an aorta of a patient; the first portion and the second portion are configured to dimensionally adjust the implantable flow restriction device to induce stenosis of the aorta distal of the at least one branch vessel of between 40% and 80% and alter blood flow through the channel into the at least one branch vessel of the aorta while maintaining a substantially unrestricted blood flow within the aorta proximal to the at least one branch vessel.

According a preferred embodiment of the invention, the implantable flow restriction device is configured to induce stenosis of the aorta distal of the at least one branch vessel of the aorta between 50% and 70%.

According to the invention, the implantable flow restriction device is configured to dimensionally adjust to alter blood flow into at least one branch vessel of the aorta.

According to an aspect not covered by the invention, the implantable flow restriction device includes a flow restricting balloon configured to dimensionally adjust to induce stenosis of the aorta distal of the at least one branch vessel of the aorta between 40% and 80%.

According to an aspect not covered by the invention, the implantable flow restriction device includes a central portion having a reduced diameter relative to at least one end portion of the implantable flow restriction device that is configured to dimensionally adjust to induce stenosis of the aorta distal of the at least one branch vessel of the aorta between 40% and 80%.

According to an aspect not covered by the invention, the implantable flow restriction device includes a main trunk portion and one or more branches extending from the main trunk portion, the one or more branches having a reduced diameter relative to the main trunk portion to dimensionally adjust the implantable flow restriction device to induce stenosis of the aorta distal of the at least one branch vessel of the aorta between 40% and 80%.

According to an aspect not covered by the invention, the implantable flow restriction device includes a restriction portion fixed within a body portion by one or more tethers, the restriction portion dimensionally adjusting the implantable flow restriction device to induce stenosis of the aorta distal of the at least one branch vessel of the aorta between 40% and 80%

According to an aspect not covered by the invention, the implantable flow restriction device includes a first adjustable portion and a second adjustable portion, and at least one of the first adjustable portion and the second adjustable portion are configured to dimensionally adjust the implantable flow restriction device to induce stenosis of the aorta distal of the at least one branch vessel of the aorta between 40% and 80%.

According to an aspect not covered by the invention, the at least one of the first adjustable portion and the second adjustable portion include a portion of decreasing dimension configured to adjust to alter blood flow therethrough or pressure distally of the implantable flow restriction device.

According to an aspect not covered by the invention, the first adjustable portion and the second adjustable portion are configured to implant on either side of the at least one branch vessel.

According to an aspect not covered by the invention, the implantable flow restriction device includes a balloon portion and a catheter portion, and the balloon portion is configured to dimensionally adjust the implantable flow restriction device to induce stenosis of the aorta distal of the at least one branch vessel of the aorta between 40% and 80%.

According to an aspect not covered by the invention, the implantable flow restriction device includes a restriction structure and a knob, the knob being configured to dimensionally adjust the restriction structure to induce stenosis of the aorta distal of the at least one branch vessel of the aorta between 40% and 80%.

According to a preferred embodiment, the implantable flow restriction device is configured to alter pressure within the aorta to alter blood flow into the at least one branch vessel of the aorta.

According to a preferred embodiment, the implantable flow restriction device is configured to increase pressure distal to the at least one branch vessel of the aorta to increase blood flow into the at least one branch vessel of the aorta.

According to one aspect not covered by the invention there is provided a method for improving cardiac function, the method includes: arranging an implantable flow restriction device with an aorta of a patient; and adjusting physical dimensions of the implantable flow restriction device to increase blood flow into at least one branch vessel of the aorta while maintaining a substantially unrestricted blood flow within the aorta proximal to the at least one branch vessel to influence renal artery pressure and regulate systemic blood pressure.

According to another aspect not covered by the invention, the implantable flow restriction device includes a flow restricting balloon and adjusting the physical dimensions of the implantable flow restriction device includes altering dimensions of the flow restricting balloon.

According to another aspect not covered by the invention, the implantable flow restriction device includes one or more branches configured to direct blood flow, and adjusting the physical dimensions of the implantable flow restriction device includes altering dimensions of at least one of the one or more branches to alter blood flow into at least one of the renal arteries.

According to another aspect not covered by the invention, adjusting the physical dimensions of the implantable flow restriction device includes manipulating a magnitude of constriction for flow through the implantable flow restriction device.

According to another aspect not covered by the invention, the at least one branch vessel of the aorta is at least one renal artery, and adjusting the physical dimensions of the implantable flow restriction device increases blood flow into the at least one of the renal artery to increase kidney perfusion hemodynamically.

According to another aspect not covered by the invention, the method also includes adjusting an amount of restriction applied by the implantable flow restriction device after implantation in response to the patient needs.

According to another aspect not covered by the invention, adjusting the physical dimensions of the implantable flow restriction device includes altering flow into kidneys of the patient to produce a neuro-hormonal response that effects a change in the patient to move toward normal kidney functioning.

According to another aspect not covered by the invention, the method also includes a sensor arranged with the implantable flow restriction device and configured to monitor blood flow therethrough, and the sensor is at least one of a pressure sensor and a flow sensor.

According to another aspect not covered by the invention, the method also includes altering the blood flow through the implantable flow restriction device in response to the blood flow monitored by the sensor.

According to an aspect not covered by the invention, there is provided a method of improving cardiac function, the method includes: arranging an implantable flow restriction device within an aorta of a patient; and adjusting physical dimensions of the implantable flow restriction device to increase blood flow into at least one branch vessel of the aorta while maintaining a substantially unrestricted blood flow within the aorta proximal to the at least one branch vessel to preserve or improve cardiac function of the patient by reducing activation of a sympathetic nervous system of the patient to decrease at least one of resting heart rate, blood pressure, and N-terminal pro b-type natriuretic peptide (nt-proBNP) or improve heart contractility.

According to another aspect not covered by the invention, adjusting the physical dimensions of the implantable flow restriction device induces stenosis of the aorta distal of the at least one branch vessel of the aorta between 40% and 80%.

According to another aspect not covered by the invention, adjusting the physical dimensions of the implantable flow restriction device induces stenosis of the aorta distal of the at least one branch vessel of the aorta between 50% and 70%.

According to another aspect not covered by the invention, the at least one branch vessel of the aorta is at least one renal artery and adjusting the physical dimensions of the implantable flow restriction device includes increasing blood pressure at an ostium of the at least one renal artery pressure across a kidney of the patient relative to venous outflow pressure causing more blood to flow through the kidney.

According to another aspect not covered by the invention, increasing the blood pressure at the ostium of the at least one renal artery includes increasing fluid filtration by the kidney to increase diuresis and lessen fluid retention of the patient.

According to another aspect not covered by the invention, increasing the blood pressure at the ostium of the at least one renal artery reduces stimulation of a Renin-Angiotensin-Aldosterone system (RAAS) of the patient.

According to another aspect not covered by the invention, there is provided a method of improving kidney function, the method includes: arranging an implantable flow restriction device with an aorta of a patient; and adjusting physical dimensions of the implantable flow restriction device to increase blood flow into at least one branch vessel of the aorta while maintaining a substantially unrestricted blood flow within the aorta proximal to the branch vessel to preserve or improve kidney health of the patient.

According to another aspect not covered by the invention, adjusting the physical dimensions of the implantable flow restriction device induces stenosis of the aorta distal of the at least one branch vessel of the aorta between 40% and 80%.

According to another aspect not covered by the invention, adjusting the physical dimensions of the implantable flow restriction device induces stenosis of the aorta distal of the at least one branch vessel of the aorta between 50% and 70%.

According to another aspect not covered by the invention, the at least one branch vessel of the aorta is at least one renal artery and adjusting the physical dimensions of the implantable flow restriction device includes increasing blood pressure at an ostium of the at least one renal artery pressure across a kidney of the patient relative to venous outflow pressure causing more blood to flow through the kidney.

According to another aspect not covered by the invention, adjusting the physical dimensions of the implantable flow restriction device to increases urine production, lowers serum creatinine, or lowers plasma NGAL.

According to an aspect of the disclosure, an apparatus includes an implantable flow restriction device configured to: implant within a vessel of a patient at a location, the vessel providing blood flow to an organ; and induce stenosis of the vessel distal of the location of between 40% and 80% and alter blood flow into the organ while maintaining a substantially unrestricted blood flow within the vessel proximal to the location.

According to another aspect of the disclosure, the implantable flow restriction device may be configured to dimensionally adjust to alter blood flow into the organ.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 shows an example implantable flow restriction device in accordance with various aspects of the present disclosure.
FIG. 2 shows an example implantable flow restriction device that includes a constraining cuff in accordance with various aspects of the present disclosure.
FIG. 3 shows an example implantable flow restriction device that includes a flow restricting stent graft in accordance with various aspects of the present disclosure.
FIG. 4A shows an example implantable flow restriction device that includes a flow diversion stent graft in accordance with various aspects of the present disclosure.
FIG. 4B shows an example implantable flow restriction device that includes another flow diversion stent graft in accordance with various aspects of the present disclosure.
FIG. 5 shows an example implantable flow restriction device that includes a flow restricting balloon in accordance with various aspects of the present disclosure.
FIG. 6 shows an example implantable flow restriction device in accordance with various aspects of the present disclosure.
FIG. 7 shows another example implantable flow restriction device in accordance with various aspects of the present disclosure.
FIG. 8A shows a perspective view illustration of an example implantable flow restriction device in accordance with the invention.
FIG. 8B shows a top view the example implantable flow restriction device, shown in FIG. 8A, in accordance with the invention.
FIG. 9 shows another example implantable flow restriction device in accordance with various aspects of the present disclosure.
FIG. 10 shows another example implantable flow restriction device in accordance with various aspects of the present disclosure.
FIG. 11 shows another example implantable flow restriction device in accordance with various aspects of the present disclosure.

While the disclosed subject matter is amenable to various modifications and alternative forms, specific embodiments have been shown by way of example in the drawings and are described in detail below. The intention, however, is not to limit the disclosure to the particular embodiments described. The invention is defined in the appended claims.

As the terms are used herein with respect to ranges of measurements (such as those disclosed immediately above), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

### DETAILED DESCRIPTION

Various aspects of the present disclosure are directed toward treating heart failure in a patient and/or other cardiovascular diseases such as hypertension and hypotension. Various aspects of the disclosure are directed toward an implantable device of manipulating renal blood flow hemodynamics in order to induce a physiologically mediated therapeutic response. In certain instances, heart function of a patient may be compromised by buildup of excess fluid (e.g., hypervolemia) in the body. The buildup of fluid may increase fluid accumulation, principally in the tissues, and increase pressure in the various circulations. The increased pressure in and of itself or in combination with an already failing heart may further harm the patient. As discussed in further detail below, various aspects of the present discourse are directed toward mitigating against buildup of excess fluid and diverting excess fluid from the heart.

In certain instances, heart function of a patient may be compromised by buildup of excess fluid (e.g., hypervolemia) in the body. The buildup of fluid may increase fluid accumulation, principally in the tissues, and increase pressure in the various circulations. The increased pressure in and of itself or in combination with an already failing heart may further harm the patient. The flow altering device, in certain instances, the increased natural diuresis lessens buildup of excess fluid and diverts excess fluid from around the heart (and/or chest cavity).

In certain instances, patients with heart failure (such as late-stage heart failure) have decreased cardiac output (e.g., amount of blood pumped by the heart per minute), which can lead to decreased diuresis. Flow restriction devices discussed herein, and the methods disclosed herein and which are not covered by the invention and that include the flow restriction devices, are directed toward increasing blood pressure at an ostium of a renal artery of a patient to increase pressure across the kidney relative to the venous outflow pressure. This may cause more blood to flow through the kidney, which enables the kidney to increase fluid filtration resulting in improved diuresis and less fluid retention.

In certain instances, patients with heart failure (such as late-stage heart failure) may have an elevated sympathetic nervous system state in part due to decreased cardiac output (blood pressure and flow in one of both of the kidneys). One compensatory output of this state is to generate a signal to attempt to preserve cardiac output, which puts further strain (myocardial oxygen demand) on the heart. Flow restriction devices discussed herein, and the methods disclosed herein and which are not covered by the invention and that include the flow restriction devices, are directed toward increasing the pressure (mean or peak systolic) in the kidney to reduce stimulation of the neuro-hormonal response (e.g., decrease in the sympathetic activation nervous system). The result of the reduced activation of the sympathetic nervous system, by way of the flow restriction device or methods that include the flow restriction device, may decrease resting heart rate and blood pressure.

Further and in certain instances, patients with heart failure (such as late-stage heart failure) may have activation of the Renin-Angiotensin-Aldosterone system (RAAS), in part due to decreased cardiac output resulting in impaired blood flow to the kidney. A consequence of the elevated RAAS is to generate a signal that stimulates adverse myocardial structural changes. Flow restriction devices discussed herein, and the methods disclosed herein and which are not covered by the invention that include the flow restriction devices, are directed toward increasing the pressure (mean or peak systolic) in the kidney to reduce stimulation of the RAAS. The result of the reduced activation of the RAAS, by way of the flow restriction device or methods that include the flow restriction device, may be a reduced sympathetic nervous system activation and attenuation of adverse cardiac remodeling.

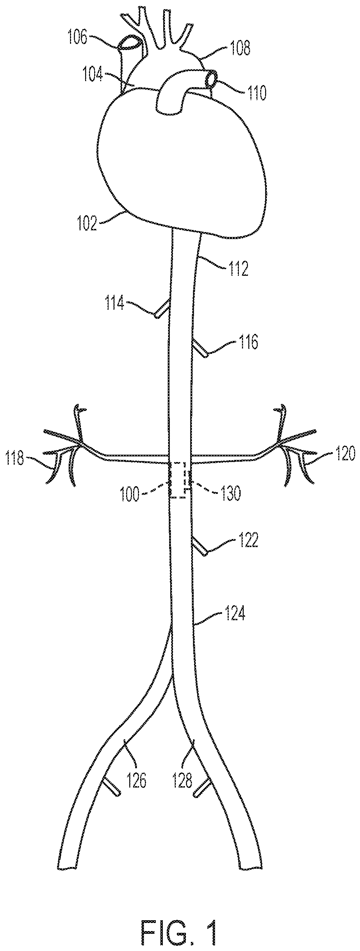

FIG. 1 shows an example implantable flow restriction device 100 in accordance with various aspects of the present disclosure. The implantable flow restriction device 100 is shown arranged within a patient's vasculature. The patient's vasculature shown in FIG. 1 includes the patient's heart 102, aortic root 104, superior vena cava 106, aortic arch 108, pulmonary trunk 110, descending aorta 112, celiac artery 114, superior mesenteric artery 116, renal arteries 118, 120, inferior mesenteric artery 122, abdominal aorta 124, and iliac arteries 126, 128. The implantable flow restriction device 100 may be arranged within the aorta distal of the renal arteries 118, 120. In addition, the implantable flow restriction device 100 may be configured to increase blood flow into at least one of the renal arteries 118, 120 while maintaining a substantially unrestricted blood flow within the aorta proximal to the renal arteries 118, 120. In addition, the implantable flow restriction device 100 may be arranged around one or both of the iliac arteries 126, 128 in addition to or alternatively of the implantable flow restriction device 100 being arranged with the aorta distal of the renal arteries 118, 120.

In certain instances, the implantable flow restriction device 100 may be for augmenting perfusion of a branch vessel (e.g., renal arteries 118, 120 or iliac arteries 126, 128) originating from the aorta. The implantable flow restriction device 100 may be adjusted by increasing resistance to blood flow through the implantable flow restriction device 100 to increase pressure within the aorta to increase blood flow into the branch vessel. In addition, the implantable flow restriction device 100 may be configured to remain with the aorta for continuously augmenting perfusion.

The implantable flow restriction device 100 being configured to increase blood flow into at least one of the renal arteries 118, 120 may divert fluid away from the heart. In a patient suffering from heart failure, fluid overload may be caused (at least in part) by insufficient blood flow through the kidneys resulting from compromised cardiac output. Use of the implantable flow restriction device 100 to increase blood flow into at least one of the renal arteries 118, 120 may increase kidney perfusion hemodynamically rather than pharmaceutically. To achieve increased kidney perfusion, resistance to blood flow distal to the renal arteries 118, 120 may be increased, which decreases distal perfusion. The increased kidney perfusion enhances renal production and therefore removes fluid volume. In addition, the implantable flow restriction device 100 may be used to enhance the performance of pharmacological treatments taken in connection therewith. For example, pharmacological treatments (e.g., diuretics and/or hypertensive medications) may be enhanced by additionally enhancing the patient's kidney function.

In certain instances, the implantable flow restriction device 100 being configured to increase blood flow into at least one of the renal arteries 118, 120 while maintaining a substantially unrestricted blood flow within the aorta proximal to the renal arteries 118, 120 may focus blood flow into the one or both of the renal arteries 118, 120. The restriction proximal to the renal arteries 118, 120 may direct blood flow to other areas supplied by the aorta such as the celiac artery 114, the superior mesenteric artery 116, or the brain. Thus, it certain instances, the implantable flow restriction device 100 may be arranged within the aorta of the patient at least partially distal of the renal arteries 118, 120. The result may be increased blood flow to at least one of the kidneys, by way of the increased blood flow to one or both of the renal arteries 118, 120, which may increase fluid removal and decrease pressure on the patient's heart.

The implantable flow restriction device 100 provides a non-pharmaceutical approach to increasing urine production and/or modifying systemic blood pressure. Patients may experience drug resistance, inaccurate dosing, or undesirable side effects. When drugs fail, aquapheresis or hemodialysis may be used to filter fluid directly from blood, however, these solutions are relatively invasive and disruptive to patient lifestyle. In addition, aquapheresis or hemodialysis may also produce hemodynamic instability with related cardiovascular complications, kidney damage, infection, and/or require capital equipment.

The implantable flow restriction device 100 may change peripheral resistance when implanted percutaneously or surgically, temporarily or permanently, and may be adjustable to meet patient needs. The implantable flow restriction device 100 may remain in the body after implantation for as long as the patient requires intervention. The implantable flow restriction device 100 may be implanted for hours, days, or even years.

The amount or resistance or flow restriction applied by the implantable flow restriction device 100 may be adjusted after implantation to meet patient needs. The implantable flow restriction device 100 may alter pressure within the aorta to increase or decrease blood flow into the renal arteries 118, 120 or targeted branch such as the iliac arteries 126, 128. An increase of pressure distal to the renal arteries 118, 120 or targeted branch such as the iliac arteries 126, 128 by the implantable flow restriction device 100 may increase blood flow into areas proximal thereto (e.g., into the renal arteries 118, 120 and/or the iliac arteries 126, 128 depending on the placement of the implantable flow restriction device 100).

In certain instances, the implantable flow restriction device 100 may produce a long-term or chronic physiological change in the patient. The implantable flow restriction device 100 altering flow into the kidneys may produce a neuro-hormonal response that effects a change in the patient to move toward normal kidney functioning. The kidneys are a feedback regulator of systemic pressure through the patient's body. The implantable flow restriction device 100 altering flow into the kidneys provides a non-pharmaceutical means of influencing the kidneys' natural feedback mechanisms to regulate systemic pressure. By adjusting the degree of hemodynamic alteration of renal perfusion, patient-specific adjustments to regulate blood pressure may be made. Adjusting the aortic flow resistance imparted by the flow restriction device 100, may influence renal artery pressure and/or flow rate, which, in turn, can manifest as transient or long-lasting alterations in systemic blood pressure. The changes induced by the flow restriction device 100, in renal-mediated blood pressure levels, may have therapeutic benefits in and of themselves. Likewise, changes induced by the flow restriction device 100 in renal-mediated blood pressure levels may be used in combination with various blood pressure medications to optimize blood pressure management on an individualized basis. In certain instances, the flow restriction device 100 may increase a resistance to blood flow, within the aorta distal to the renal arteries 118, 120, by approximately 10% to 30% as compared to normal flow. The flow restriction device 100 may occlude the aorta distal to the renal arteries 118, 120 by approximately 10% to 30 to increase blood flow blood flow into the kidneys. In certain instances, occluding the aorta distal to the renal arteries 118, 120 (increasing resistance to blood flow therethrough) at a percentage greater than approximately 70%, may decrease blood flow to the kidneys based on the kidneys' natural feedback mechanisms to regulate systemic pressure.

In certain instances, the implantable flow restriction device 100 may include a sensor 130. The sensor 130 may be configured to monitor blood flow (or other hemodynamic parameters) in or near the implantable flow restriction device 100. The sensor 130 may, alternatively, monitor various biochemical, biomarker, or pharmacological parameters in the blood stream. The sensor 130 may be one of a pressure sensor or a flow sensor. In addition, the implantable flow restriction device 100 may be configured to alter the amount of blood flow in response to the blood flow monitored by the sensor 130. In certain instances, the implantable flow restriction device 100 may be configured to alter blood flow to one or more of the renal arteries 118, 120 by altering a pressure differential within the aorta or within the renal arteries 118, 120. The implantable flow restriction device 100 altering the pressure differential may increase or decrease blood flow into one or both of the renal arteries 118, 120. For example, the increase in pressure by the implantable flow restriction device 100 distal to the renal arteries 118, 120 will force blood into the renal arteries 118, 120. The amount of pressure applied by the implantable flow restriction device 100 may be adjusted to achieve a desired blood flow into one or both of the renal arteries 118, 120. The sensor 130 may be used in combination with sources of information external to the patient such as personal health trackers or lifestyle coaching interfaces to assist in the amount of pressure applied by the implantable flow restriction device 100 to achieve a desired blood flow into one or both of the renal arteries 118, 120.

In certain instances, the implantable flow restriction device 100 is configured to induce stenosis of the aorta of the patient at least partially distal of the renal arteries 118, 120 between 40% and 80% and alter blood flow into at least one branch vessel of the aorta (e.g., one or both of the renal arteries 118, 120) while maintaining a substantially unrestricted blood flow within the aorta proximal to the at least one branch vessel (e.g., one or both of the renal arteries 118, 120). In certain instances, the induced stenosis is between 50% and 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion. In addition, the implantable flow restriction device 100 may be implanted into another vessel of the patient that leads into an organ. In these instances, the implantable flow restriction device 100 is configured to induce stenosis of the vessel into which the implantable flow restriction device 100 distal of location at which the implantable flow restriction device 100 is implanted between 40% and 80%. In addition, implanting the implantable flow restriction device 100 in this manner alter blood flow into the organ that the vessel leads into while maintaining a substantially unrestricted blood flow within the vessel proximal to the location of implantation.

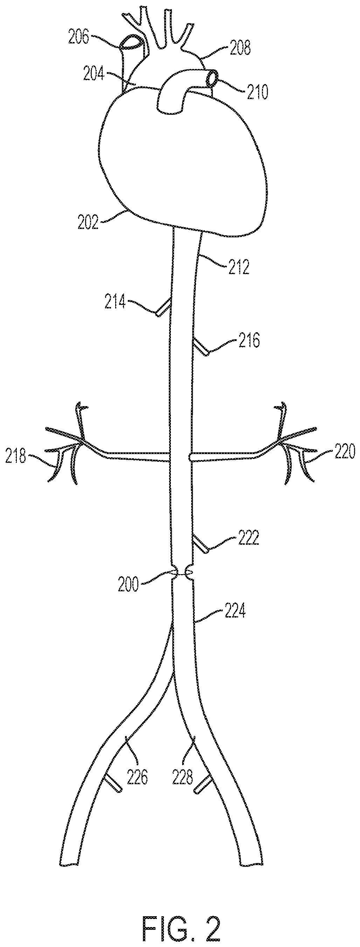

In addition and in certain instances, the implantable flow restriction device 100 may be used for improving cardiac function by adjusting physical dimensions of the implantable flow restriction device 100 to increase blood flow into at least one branch vessel of the aorta (e.g., the renal arteries 118, 120) while maintaining a substantially unrestricted blood flow within the aorta proximal to the branch vessel to preserve or improve cardiac function of the patient. For further discussion of adjusting physical dimensions, reference may be made to FIGs. 2-11.

Adjusting the physical dimensions of the implantable flow restriction device 100 may induce stenosis of the aorta distal of the at least one branch vessel (e.g., the renal arteries 118, 120) of the aorta between 40% and 80% and in certain more specific instances, between 50% and 70%. In certain instances, adjusting the physical dimensions of the implantable flow restriction device 100 includes increasing blood pressure at an ostium of at least one of the renal arteries 118, 120 to increase pressure across a kidney of the patient relative to venous outflow pressure causing more blood to flow through the kidney. In addition, increasing the blood pressure at the ostium of at least one of the renal arteries 118, 120 includes increasing fluid filtration by the kidney to increase diuresis and lessen fluid retention of the patient. Further, increasing the blood pressure at the ostium of at least one of the renal arteries 118, 120 reduces activation of a sympathetic nervous system of the patient to decrease resting heart rate, blood pressure, and/or N-terminal pro b-type natriuretic peptide (nt-proBNP). In addition, reducing activation of the sympathetic nervous system may also improve heart contractility. In certain instances, increasing the blood pressure at the ostium of at least one of the renal arteries 118, 120 reduces stimulation of a Renin-Angiotensin-Aldosterone system (RAAS) of the patient.

In certain instances, the implantable flow restriction device 100 may be used to increase blood flow into at least one branch vessel of the aorta while maintaining a substantially unrestricted blood flow within the aorta proximal to the branch vessel to preserve or improve kidney health of the patient by adjusting physical dimensions of the implantable flow restriction device 100. Kidney health may include the amount of injury that the kidney has sustained, is continuing to sustain, or a decrease in function relative to the baseline kidney function of a patient when healthy. In certain instances, kidney injury may be quantified by measuring Neutrophil gelatinase-associated lipocalin (NGAL). For further discussion of adjusting physical dimensions, reference may be made to FIGs. 2-11. Adjusting the physical dimensions of the implantable flow restriction device 100 may induce stenosis of the aorta distal of the at least one branch vessel (e.g., the renal arteries 118, 120) of the aorta between 40% and 80% and in certain more specific instances, between 50% and 70%. Clinically, measurement of ankle pressure, Doppler ultrasound velocity, or other hemodynamic parameters in the lower limbs can be employed to optimize the magnitude of the induced stenosis while ensuring adequate limb perfusion.

The illustrative implantable flow restriction device 100 shown in FIG. 1 is not intended to suggest any limitation as to the scope of use or functionality of embodiments of the disclosure disclosed throughout this document. Neither should the illustrative implantable flow restriction device 100 be interpreted as having any dependency or requirement related to any single component or combination of components illustrated therein. Additionally, any one or more of the components depicted in FIG. 1 can be, in embodiments, integrated with various ones of the other components depicted therein (and/or components not illustrated). For example, flow restriction devices discussed with reference to FIGS. 2-11 may include a sensor as discussed with reference to FIG. 1.

FIG. 2 shows an example implantable flow restriction device 200 that includes a constraining cuff in accordance with various aspects of the present disclosure. The implantable flow restriction device 200 is shown arranged with a patient's aorta. The patient's vasculature shown in FIG. 2 includes the patient's heart 202, aortic root 204, superior vena cava 206, aortic arch 208, pulmonary trunk 210, descending aorta 212, celiac artery 214, superior mesenteric artery 216, renal arteries 218, 220, inferior mesenteric artery 222, abdominal aorta 224, and iliac arteries 226, 228. The implantable flow restriction device 200 may arranged with the aorta distal of the renal arteries 218, 220.

The implantable flow restriction device 200 may be configured to increase blood flow into at least one of the renal arteries 218, 220. As shown in FIG. 2, the implantable flow restriction device 200 may be a constraining cuff. In certain instances, the constraining cuff may be a suture, a thin graft layer (e.g., expanded polytetrafluoroethylene (ePTFE)), or other similar structure. Arranging the implantable flow restriction device 200 may include arranging the constraining cuff around the aorta distal of the renal arteries 218, 220 to increase blood flow into at least one of the renal arteries 218, 220. In addition, the implantable flow restriction device 200 may be arranged around one or both of the iliac arteries 226, 228 in addition to or alternatively of the implantable flow restriction device 200 being arranged around the aorta distal of the renal arteries 218, 220.

The constraining cuff implantable flow restriction device 200 may reduce a diameter of the aorta and/or iliac arteries 226, 228 to increase resistance and reduce blood flow distally thereof. The amount of diameter reduction of the constraining cuff implantable flow restriction device 200 may be adjusted to optimize flow to the kidneys via at least one of the renal arteries 218, 220 while not compromising distal perfusion to the point of producing significant clinical symptoms. The amount of diameter reduction of the constraining cuff implantable flow restriction device 200 may be altered by adjusting the tension applied via the constraining cuff implantable flow restriction device 200 to alter blood flow into at least one of the renal arteries 218, 220.

The constraining cuff implantable flow restriction device 200 may also maintain a substantially unrestricted blood flow within the aorta proximal to the renal arteries 218, 220. The constraining cuff implantable flow restriction device 200 being located distal to the arteries 218, 220 may increase blood flow therein, while maintaining a substantially normal flow to areas of the aorta proximal to the renal arteries 218, 220. Blood flow may be monitored proximal to the renal arteries 218, 220 and the amount of restriction applied via the constraining cuff implantable flow restriction device 200 may be adjusted to maintain a substantially normal flow to areas of the aorta proximal to the renal arteries 218, 220 and also to increase blood flow into at least one of the renal arteries 218, 220.

The amount or resistance or flow restriction applied by the constraining cuff implantable flow restriction device 200 may be adjusted after implantation to meet patient needs. The constraining cuff implantable flow restriction device 200 may alter pressure within the aorta to increase or decrease blood flow into the renal arteries 218, 220 or targeted branch such as the iliac arteries 226, 228. An increase of pressure distal to the renal arteries 218, 220 or targeted branch such as the iliac arteries 226, 228 by the constraining cuff implantable flow restriction device 200 may increase blood flow into areas proximal thereto (e.g., into the renal arteries 218, 220 and/or the iliac arteries 226, 228 depending on the placement of the constraining cuff implantable flow restriction device 200). In certain instances, the constraining cuff implantable flow restriction device 200 may produce a long-term or chronic physiological change in the patient. The constraining cuff implantable flow restriction device 200 altering flow into the kidneys may produce a neuro-hormonal response that effects a change in the patient to move toward normal kidney functioning. The kidneys are a feedback regulator of systemic pressure through the patient's body. The implantable flow restriction device 100 altering flow into the kidneys provides a feedback mechanism through the body for systemic pressure, and may be adjustable to meet patient needs.

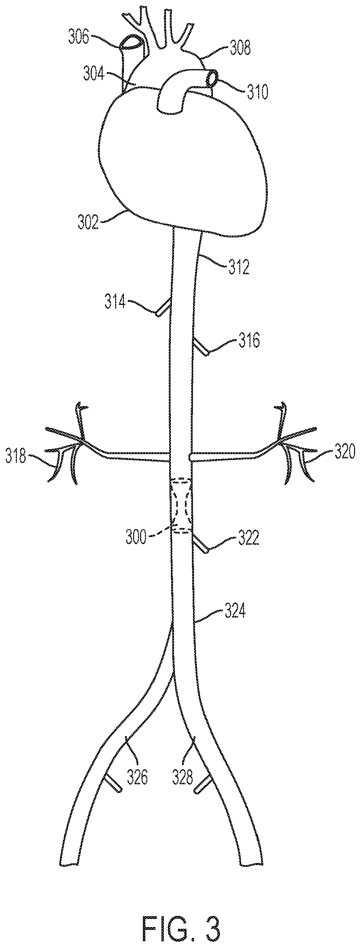

FIG. 3 shows an example implantable flow restriction device 300 that includes a flow restricting stent graft in accordance with various aspects of the present disclosure. The implantable flow restriction device 300 is shown arranged with a patient's aorta. The patient's vasculature shown in FIG. 3 includes the patient's heart 302, aortic root 304, superior vena cava 306, aortic arch 308, pulmonary trunk 310, descending aorta 312, celiac artery 314, superior mesenteric artery 316, renal arteries 318, 320, inferior mesenteric artery 322, abdominal aorta 324, and iliac arteries 326, 328. The implantable flow restriction device 300 may arranged with the aorta distal of the renal arteries 318, 320.

The implantable flow restriction device 300 may be configured to increase blood flow into at least one of the renal arteries 318, 320. As shown in FIG. 3, the implantable flow restriction device 300 may be a flow restricting stent graft. The flow restricting stent graft device 300 may be configured to reduce a diameter of the aorta and/or the iliac arteries 326, 328 to increase resistance and reduce blood flow distally thereof. Although FIG. 3 shows the flow restricting stent graft device 300 arranged within the aorta, a patient may additionally or alternatively include the flow restricting stent graft device 300 arranged within one or both of the iliac arteries 326, 328. The flow restricting stent graft device 300 may be diametrically adjustable. The restricting stent graft device 300 may be diametrically adjusted multiple times to achieve a desired shape and diameter(s). In certain instances, the flow restricting stent graft device 300 may be diametrically adjustable by way of a distensible force applied within the flow restricting stent graft device 300. The distensible force may be applied by way of a balloon structure. The flow restricting stent graft device 300 may include a stent and a graft structure. Either or both of the stent and the graft structure of the flow restricting stent graft device 300 may be configured to maintain a new diameter in response to the distensible force.

In certain instances, the flow restricting stent graft device 300 may include stent components that are expandable, for example, by balloon. The flow restricting stent graft device 300 may include expandable portions or the entire flow restricting stent graft device 300 may be balloon expandable. For example, expandable portions of the flow restricting stent graft device 300 may be configured to have controlled expansion that allows diametric adjustment beyond an initial deployment diameter through a plurality of adjusted diameters up to a maxim diametric expansion limit of the balloon expandable flow restricting stent graft device 300. Examples of diametrically adjustable devices and associated methods are also described in U.S. Patent Publication 2016/0143759 to Bohn et al.

Examples of suitable stent patterns and associated methods of manufacture are also described in U.S. Patent 6,673,102 to Vonesh et al. Stents of the flow restricting stent graft device 300 may be formed from a variety of wire materials, including stainless steel, nickel-titanium alloy (nitinol), tantalum, elgiloy, various polymer materials, such as poly(ethylene terephthalate) (PET) or polytetrafluoroethylene (PTFE), or bioresorbable materials, such as levorotatory polylactic acid (L-PLA) or polyglycolic acid (PGA). In various examples, the stent or stents are self-expanding and exert a self-expansion force on the flow restricting stent graft device 300 when constrained. As such, the stent or stents of the flow restricting stent graft device 300 may be formed of superelastic materials, such as nitinol metal, that will withstand tight compression in a compacted configuration (diameter) and then self-expand to a deployed configuration (diameter) once released in place, such as those described in U.S. Patent 6,673,102 to Vonesh et al. In some embodiments, the flow restricting stent graft device 300 may be configured to be mechanically adjust under pressure greater than typical biological pressures (e.g., typically circulatory pressures) and any expansion force exerted by the stent of the flow restricting stent graft device 300.

For example, the flow restricting stent graft device 300 may have a controlled expansion element that is optionally mechanically adjustable by causing controlled expansion material forming one or more portions of the flow restricting stent graft device 300 to yield or plastically deform, by causing reorganization of a fibrillary or other microstructure of such controlled expansion material, by release of fasteners or folds of the flow restricting stent graft device 300, or other mechanical adjustment of the flow restricting stent graft device 300. The pressure required to mechanically adjust the flow restricting stent graft device 300 is greater than typical physiologic conditions (e.g., typical maximum blood pressures) such that the flow restricting stent graft device 300 is able to maintain the adjusted diameter at less than a pressure that would tend to cause the flow restricting stent graft device 300 to catastrophically fail by exceeding the maximum diametric expansion limit of the flow restricting stent graft device 300. The controlled expansion of the flow restricting stent graft device 300 is preferably configured to maintain a diameter to which it is mechanically adjusted without substantial diameter creep or spontaneous diametric expansion over time under typical biological conditions. The controlled expansion portions of the flow restricting stent graft device 300 optionally includes one or more layers and may be formed from a variety of materials, including fluoropolymer materials such as the distensible, expanded PTFE tube described in U.S. Patents 3,953,556, 3,962,153, 4,096,227, 4,187,390, and 4,902,423, to Gore or the distensible lattices of U.S. 2013/0204347 to Armstrong, et al. For further reference regarding controlled expansion, reference may be made to PCT/US2016/028671, filed April 21, 2016.

The flow restricting stent graft device 300 may be an hour-glass shaped stent-graft. The hour-glass shaped flow restricting stent graft device 300 may have separated portions that are diametrically adjustable and include stents on each portion of the hour-glass shape. The stents may be discrete rings or portions on each section of the hour-glass shaped flow restricting stent graft device 300. For example, the larger end portions of the hour-glass shaped flow restricting stent graft device 300 may have different stent portions that the smaller diameter interior portions of the hour-glass shaped flow restricting stent graft device 300. The flow restricting stent graft device 300 may be fabricated of Nitinol (NiTi) or stainless steel and ePTFE, and may be self-expanding or balloon expandable. The hour-glass shaped flow restricting stent graft device 300 may reduce a dimension (diameter) of the aorta and/or the iliac arteries 326, 328 to increase resistance and reduce blood flow distally thereof. The hour-glass shaped flow restricting stent graft device 300 thereby increases blood flow into one or both of the renal arteries 318, 320. The amount of restriction may be adjusted, as noted above, by altering dimensions (such as the diameter) of the hour-glass shaped flow restricting stent graft device 300. The central portion of the hour-glass shaped flow restricting stent graft device 300 may be ballooned open further to adjust the flow therethrough.

The flow restricting stent graft device 300 may also maintain a substantially unrestricted blood flow within the aorta proximal to the renal arteries 318, 320. Blood flow may be monitored proximal to the renal arteries 318, 320 and the amount of restriction applied via the flow restricting stent graft device 300 may be adjusted to maintain a substantially normal flow to areas of the aorta proximal to the renal arteries 318, 320 and also to increase blood flow into at least one of the renal arteries 318, 320. In a patient suffering from heart failure, fluid overload may be caused (at least in part) by insufficient blood flow through the kidneys resulting from compromised cardiac output. The implantable flow restriction device 300 increasing blood flow into at least one of the renal arteries 318, 320 may increase kidney perfusion hemodynamically. The increased kidney perfusion may remove up to approximately one liter of excess fluid per day.

The amount or resistance or flow restriction applied by the implantable flow restriction device 300 may be adjusted after implantation to meet patient needs. The implantable flow restriction device 300 may alter pressure within the aorta to increase or decrease blood flow into the renal arteries 318, 320 (and/or the iliac arteries 326, 328). In certain instances, the implantable flow restriction device 300 may produce a long-term or chronic physiological change in the patient. The implantable flow restriction device 300 altering flow into the kidneys may produce a neuro-hormonal response that effects a change in the patient to move toward normal kidney functioning. The kidneys are a feedback regulator of systemic pressure through the patient's body. The implantable flow restriction device 300 altering flow into the kidneys provides a feedback mechanism through the body for systemic pressure, and may be adjustable to meet patient needs.

The illustrative flow restricting stent graft device 300 shown in FIG. 3 is not intended to suggest any limitation as to the scope of use or functionality of embodiments of the disclosure disclosed throughout this document. Neither should the illustrative flow restricting stent graft device 300 be interpreted as having any dependency or requirement related to any single component or combination of components illustrated therein. For example, in embodiments, the illustrative flow restricting stent graft device 300 may include aspects that allow for constriction after implantation, similar to the constraining cuff implantable flow restriction device 200. Additionally, any one or more of the components depicted in FIG. 3 can be, in embodiments, integrated with various ones of the other components depicted therein (and/or components not illustrated).

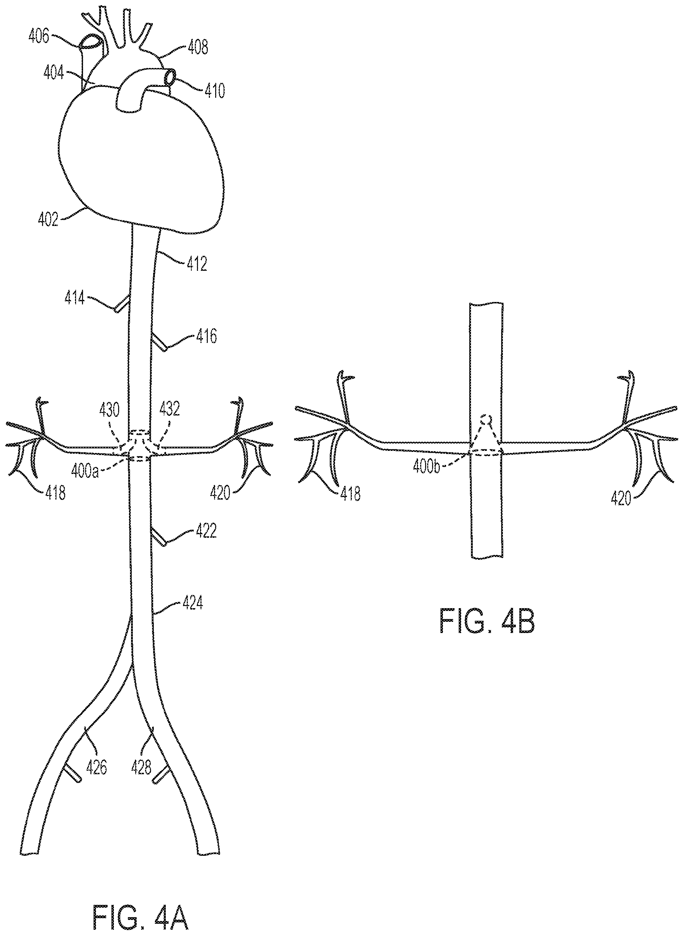

FIG. 4A shows an example flow restriction device that includes a flow diversion stent graft 400a in accordance with various aspects of the present disclosure. The implantable flow restriction device 400a is shown arranged with a patient's aorta. The patient's vasculature shown in FIG. 4A includes the patient's heart 402, aortic root 404, superior vena cava 406, aortic arch 408, pulmonary trunk 410, descending aorta 412, celiac artery 414, superior mesenteric artery 416, renal arteries 418, 420, inferior mesenteric artery 422, abdominal aorta 424, and iliac arteries 426, 428. The implantable flow restriction device 400a may be arranged with the aorta distal of the renal arteries 418, 420.

The implantable flow restriction device 400a may be configured to increase blood flow into at least one of the renal arteries 418, 420. As shown in FIG. 4, the implantable flow restriction device 400a may be a flow diversion stent graft device. The flow diversion stent graft device 400a may include one or more branches 430, 432. The branches 430, 432 may be arranged within the renal arteries 418, 420, with a main body portion of the flow diversion stent graft device 400a being arranged within the aorta. The branches 430, 432 of the flow diversion stent graft device 400a may direct blood flow into the renal arteries 418, 420 to increase blood flow therein. The flow diversion stent graft device 400a allows for blood flow distally to the flow diversion stent graft device 400a. The branches 430, 432 of the flow diversion stent graft device 400a may increase resistance for blood flow distally of the flow diversion stent graft device 400a thereby increasing blood into the renal arteries 418, 420.

In certain instances, the flow diversion stent graft device 400a may be diametrically adjustable. In certain instances, the flow diversion stent graft device 400a may be diametrically adjustable by way of a distensible force applied within the flow restricting stent graft device 400a. The force may be applied within the main body portion of the flow diversion stent graft device 400a or within the branches 430, 432 to adjust the dimensions. Dimensions of the branches 430, 432 or other portions of the flow diversion stent graft device 400a may be restricted or expanded to adjust flow. The flow diversion stent graft device 400a may be fabricated of Nitinol (NiTi) or stainless steel and ePTFE, and may be self-expanding or balloon expandable.

The flow diversion stent graft device 400a may also maintain a substantially unrestricted blood flow within the aorta proximal to the renal arteries 418, 420. Blood flow may be monitored proximal to the renal arteries 418, 420 and the amount of restriction applied via the flow diversion stent graft device 400a may be adjusted to maintain a substantially normal flow to areas of the aorta proximal to the renal arteries 418, 420 and also to increase blood flow into at least one of the renal arteries 418, 420.

FIG. 4B shows a portion of the patient's vasculature, including the renal arteries 418, 420, with another implantable flow restriction device 400b arranged within the aorta. The implantable flow restriction device 400b may also be a flow diversion stent graft device 400b without branches. The flow diversion stent graft device 400b may be a cone-shaped stent-graft. The flow diversion stent graft device 400b may force or direct blood flow into one or both of the renal arteries 418, 420. The proximal end of the flow diversion stent graft device 400b may be enlarged to increase distal flow as needed. Portions of the flow diversion stent graft device 400b may be porous to allow adequate flow through the material to prevent thrombosis. In certain instances, the flow diversion stent graft device 400b may be substantially cylindrical rather than cone shaped with diversion of blood flow into one or both of the renal arteries 418, 420 via perfusion provided by the porosity of the flow diversion stent graft device 400b.

The illustrative flow diversion stent graft devices 400a-b shown in FIG. 4A-B are not intended to suggest any limitation as to the scope of use or functionality of embodiments of the disclosure disclosed throughout this document. Neither should the illustrative flow restricting flow diversion stent graft devices 400a-b be interpreted as having any dependency or requirement related to any single component or combination of components illustrated therein. For example, in some embodiments, the illustrative flow diversion stent graft devices 400a-b may include aspects that allow for constriction after implantation, similar to the constraining cuff implantable flow restriction device 200. Additionally, any one or more of the components depicted in FIG. 4A-B can be, in some embodiments, integrated with various ones of the other components depicted therein (and/or components not illustrated). In a patient suffering from heart failure, fluid overload may be caused (at least in part) by insufficient blood flow through the kidneys resulting from compromised cardiac output. The flow restriction devices 400a-b increase blood flow into at least one of the renal arteries 418, 420, which increases kidney perfusion mechanically. The increased kidney perfusion may remove up to approximately one liter of excess fluid per day.

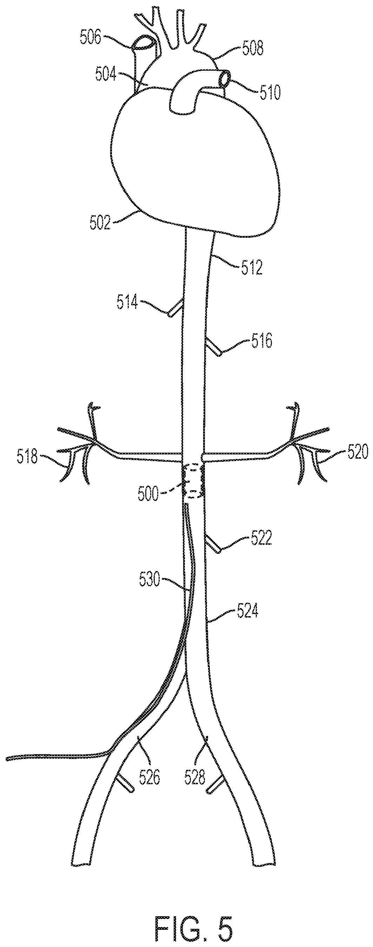

FIG. 5 shows an example implantable flow restriction device 500 that includes a flow restricting balloon in accordance with various aspects of the present disclosure. The implantable flow restriction device 500 is shown arranged with a patient's aorta. The patient's vasculature shown in FIG. 5 includes the patient's heart 502, aortic root 504, superior vena cava 506, aortic arch 508, pulmonary trunk 510, descending aorta 512, celiac artery 514, superior mesenteric artery 516, renal arteries 518, 520, inferior mesenteric artery 522, abdominal aorta 524, and iliac arteries 526, 528. The implantable flow restriction device 500 may arranged with the aorta distal of the renal arteries 518, 520.

The implantable flow restriction device 500 may be configured to increase blood flow into at least one of the renal arteries 518, 520. As shown in FIG. 5, the implantable flow restriction device 500 may be a balloon with a flow lumen 530. The balloon implantable flow restriction device 500 may decrease blood flow in an area distal of the renal arteries 518, 520 and create resistance to distal perfusion thereby forcing additional blood flow into the kidneys. The increased blood flow to at least one of the kidneys, by way of the increased blood flow to one or both of the renal arteries 518, 520, may increase fluid removal thereby decreasing pressure on the patient's heart. Altering dimensions of the balloon implantable flow restriction device 500, by increasing or decreasing the inflation thereof via the flow lumen 530, may alter blood flow into at least one of the renal arteries 518, 520. The balloon implantable flow restriction device 500 may also be detachable such that the flow lumen 530 may be removed and the balloon implantable flow restriction device 500 may be temporarily implanted in the aorta.

In addition, the balloon implantable flow restriction device 500 may also maintain a substantially unrestricted blood flow within the aorta proximal to the renal arteries 518, 520. Blood flow may be monitored proximal to the renal arteries 518, 520 and the amount of restriction applied via the balloon implantable flow restriction device 500 may be adjusted to maintain a substantially normal flow to areas of the aorta proximal to the renal arteries 518, 520 and also to increase blood flow into at least one of the renal arteries 518, 520.

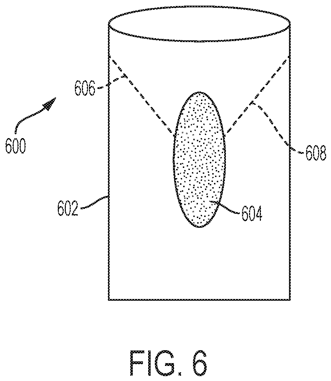

FIG. 6 shows an example implantable flow restriction device 600 in accordance with various aspects of the present disclosure. The example flow restriction device 600 may include a body portion 602 and a restriction portion 604. The example implantable flow restriction device 600 may configured to implant with an aorta of a patient and alter blood flow into at least one branch vessel of the aorta while maintaining a substantially unrestricted blood flow within the aorta proximal to the branch vessel as is described in further detail above with reference to, for example, FIG. 1.

The implantable flow restriction device 600 may include a restriction portion 604 may be arranged within the body portion 602. The restriction portion 604 may be fixed within the body portion 602 by one or more tethers 606, 608 or other similar structures. The restriction portion 604 may be arranged centrally within the body portion 602 (as shown in FIG. 6) or arranged near an internal boundary thereof. The restriction portion 604 may be configured to resist blood flow distally of the implantable flow restriction device 600 and direct flow into a branch vessel (as described in detail above). The amount of restriction, and thus blood flow, may be altered by changing a diameter of the restriction portion 604 by shortening the implantable flow restriction device 600 (increasing diameter of the restriction portion 604) or by applying a twisting motion to the body portion 602 to increase diameter, or altering flow patterns. The body portion 602 may be formed of a graft component and/or a stent component. In certain instances, the flow restriction device 600 may be used in connection with an implantable stent graft.

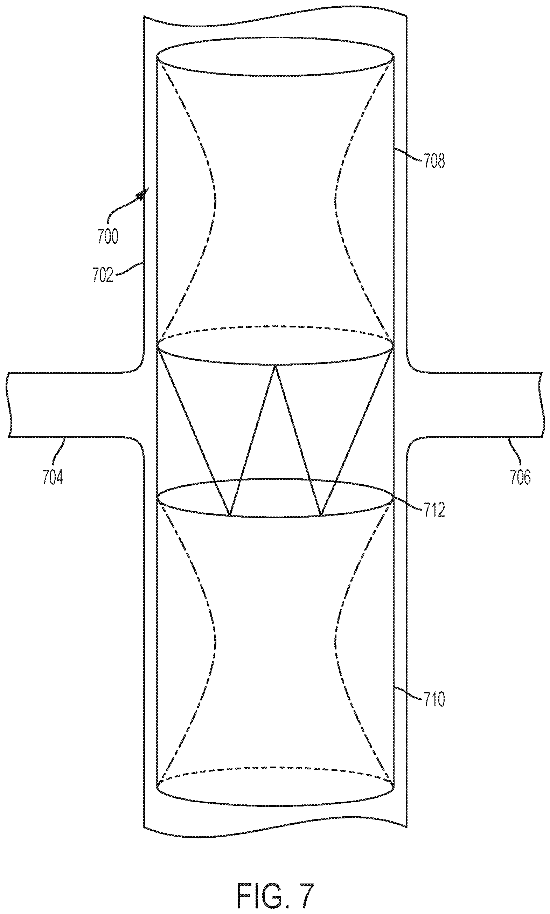

FIG. 7 shows another example implantable flow restriction device 700 in accordance with various aspects of the present disclosure. The implantable flow restriction device 700 may configured to implant with an aorta 702 of a patient and alter blood flow into at least one branch vessel 704, 706 of the aorta 702 while maintaining a substantially unrestricted blood flow within the aorta 702 proximal to the at least one branch vessel 704, 706 as is described in further detail above with reference to, for example, FIG. 1.

The implantable flow restriction device 700 may include a first adjustable portion 708 and a second adjustable portion 710. The first adjustable portion 708 and the second adjustable portion 710 may be separate by an intermediate portion 712. The intermediate portion 712 may provide structural stability for the implantable flow restriction device 700 and may include an open wire or strut structure formed by a stent-like component. Remaining portions of the implantable flow restriction device 700 may be formed by a graft component alone or in combination with a stent structure.

The first adjustable portion 708 and the second adjustable portion 710 may be configured to be implanted on either side of the at least one branch vessel 704, 706. In addition, the first adjustable portion 708 and the second adjustable portion 710 may be diametrically adjustable (e.g., balloon dilation) to alter blood flow therethrough. The first adjustable portion 708 and the second adjustable portion 710 may be funnel shaped or otherwise include a portion of decreasing dimension that may be adjusted to alter blood flow therethrough or pressure distally of the implantable flow restriction device 700.

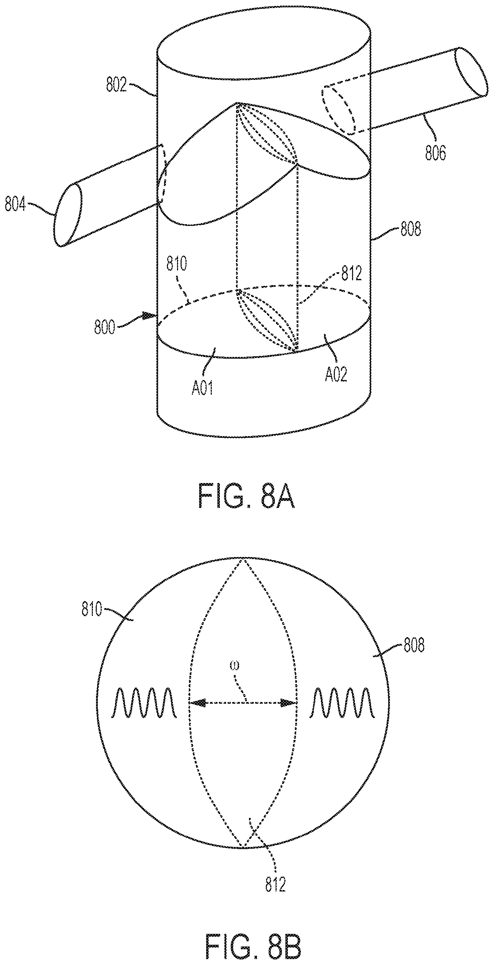

FIG. 8A-B shows a perspective view illustration and a top view of an example implantable flow restriction device 800 in accordance with various aspects of the present disclosure. The implantable flow restriction device 800 may configured to implant with an aorta 802 of a patient and alter blood flow into at least one branch vessel 804, 806 of the aorta 802 while maintaining a substantially unrestricted blood flow within the aorta 802 proximal to the at least one branch vessel 804, 806 as is described in further detail above with reference to, for example, FIG. 1. In certain instances, the implantable flow restriction device 800 may be implanted within the aorta 802 distally of the at least one branch vessel 804, 806.

The implantable flow restriction device 800 includes a first portion 808, a second portion 810, and a channel 812 therebetween. The first portion 808 and the second portion 810 are diametrically adjustable (*e.g*., balloon dilation) to alter blood flow through the channel 812. The first portion 808 and/the second portion 810 may be adjusted to alter blood flow through the channel 812 or pressure distally of the implantable flow restriction device 800.

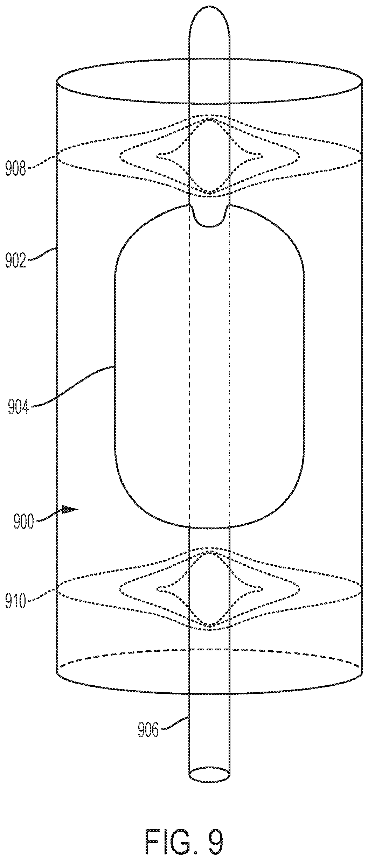

FIG. 9 shows another example implantable flow restriction device 900 in accordance with various aspects of the present disclosure. The implantable flow restriction device 900 may configured to implant with an aorta 902 of a patient and alter blood flow into at least one branch vessel (not shown) of the aorta 902 while maintaining a substantially unrestricted blood flow within the aorta 902 proximal to the at least one branch vessel as is described in further detail above with reference to, for example, FIG. 1.

The implantable flow restriction device 900 may include a balloon portion 904 and a catheter portion 906. The catheter portion 906 may include a lumen to inflate and deflate the balloon portion 904. The balloon portion 904 may be adjusted to alter blood flow through the implantable flow restriction device 900 or pressure distally of the implantable flow restriction device 900. In addition, the implantable flow restriction device 900 may include one or more stent portions 908, 910 that are configured to stabilize the implantable flow restriction device 900 within the aorta 902.

The graft components discussed herein may be made up of any material which is suitable for use as a graft in the chosen body lumen. The graft components may be composed of the same or different materials. Furthermore, the graft components may include multiple layers of material that can be the same material or different material. Many graft materials are known, particularly known are those that can be used as vascular graft materials. In one embodiment, said materials can be used in combination and assembled together to comprise a graft. The graft materials used in a stent graft can be extruded, coated or formed from wrapped films, or a combination thereof. Polymers, biodegradable and natural materials can be used for specific applications.

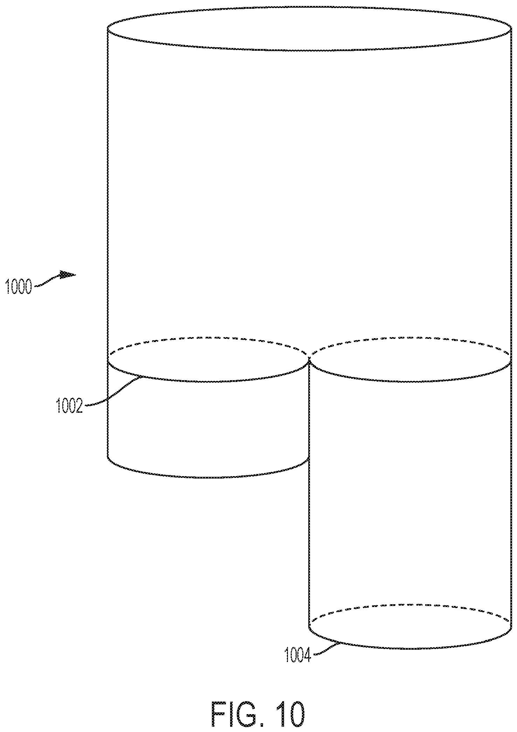

FIG. 10 shows another example implantable flow restriction device 1000 in accordance with various aspects of the present disclosure. The implantable flow restriction device 1000 may be a bifurcated stent graft that includes a first leg 1002 and a second leg 1004. The diameter and/or length of first leg 1002 and the second leg 1004 may be independently adjustable to alter resistance to blood flow through the implantable flow restriction device 1000.

The implantable flow restriction device 1000 may be implanted in a patient's aorta. The implantable flow restriction device 1000 may be implanted distal to the renal arteries to increase blood flow to the kidneys. The implantable flow restriction device 1000 may increase resistant to blood flow or pressure distal to the renal arteries while maintaining approximately nominal blood flow and pressure proximal to the implanted distal to the renal arteries.

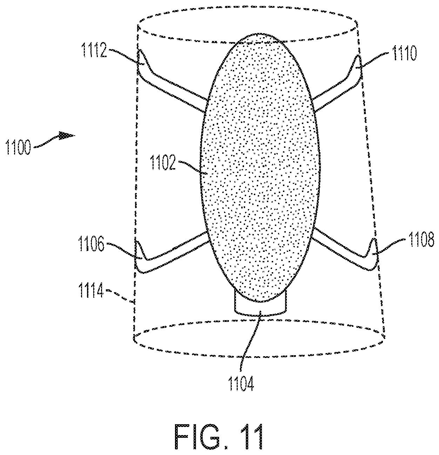

FIG. 11 shows another example implantable flow restriction device 1100 in accordance with various aspects of the present disclosure. The flow restriction device 1100 may include a restriction structure 1102. The restriction structure 1102 may be elliptical in shape and may be adjustable to increase or decrease length and/or diameter thereof. The flow restriction device 1100 may be arranged centrally within a patient's aorta. The implantable flow restriction device 1100 may be implanted in a patient's aorta. The implantable flow restriction device 1100 may be implanted distal to the renal arteries to increase blood flow to the kidneys. The implantable flow restriction device 1100 may increase resistant to blood flow or pressure distal to the renal arteries while maintaining approximately nominal blood flow and pressure proximal to the implanted distal to the renal arteries.

The restriction structure 1102 may include a knob 1104 configured to increase or decrease length and/or diameter thereof. The knob 1104 may be actuated by a catheter or wire access mechanism. The restriction structure 1102 may include a stent component and a graft component. The restriction structure 1102 may be a Nitinol structure covered by ePTFE. The stent component (Nitinol structure) of the restriction structure 1102 may be twisted based on actuation of the knob 1104 to increase or decrease length and/or diameter thereof.

The implantable flow restriction device 1100 may include anchors 1106, 1108, 1110, 1112. The anchors 1106, 1108, 1110, 1112 may be coupled to the restriction structure 1102. In addition, the anchors 1106, 1108, 1110, 1112 may be configured to anchor the implantable flow restriction device 1100 within the aorta. In certain instances, the implantable flow restriction device 1100 may include a stent-graft component 1114. The stent-graft component 1114 may facilitate removal of the implantable flow restriction structure 1102 (and anchors 1106, 1108, 1110, 1112) to be removed from the patient. Removal of the implantable flow restriction structure 1102 (and anchors 1106, 1108, 1110, 1112) while leaving behind the stent-graft component 1114 may allow for the implantable flow restriction structure 1102 to be replaced or the implantable flow restriction structure 1102 to be removed permanently.

The illustrative flow diversion stent graft devices discussed herein and shown in FIGs. 1-11 are not intended to suggest any limitation as to the scope of use or functionality of embodiments of the disclosure disclosed throughout this document. Neither should the illustrative flow restricting flow diversion stent graft devices interpreted as having any dependency or requirement related to any single component or combination of components illustrated therein. For example, in some embodiments, the illustrative flow diversion stent graft devices may include diametrically adjustable stent and/or graft components. For example, the stent components, discussed herein, may be diametrically adjustable such that elongation of the device may increase a diameter of the stent component, and shortening of the device may decrease a diameter of the stent component. For further discussion regarding diametrically adjustable stent components, reference may be made to U.S. Patent No. 8,936,634. In addition, the graft components discussed herein may also be diametrically adjustable. For further discussion regarding diametrically adjustable graft components, reference may be made to U.S. Patent No. 6,336,937 and U.S. Patent No. 9,522,072. In addition, the flow diversion stent graft devices may alter flow into the kidneys and produce a neuro-hormonal response that effects a change in the patient as discussed in further detail with reference to FIG. 1.

### Test Methods

A non-limiting example of a suitable test method for the flow restriction devices and methods that include the flow restriction devices discussed herein involved evaluating response of canines with induced heart failure (coronary microembolization resulting in ejection fraction of about 30%). Four canines received flow restricting devices distal to the renal arteries and three were kept for controls. Average diameter stenosis induced by the devices was about 60% (range of about 55-64%). Animals were kept in life for 35 days; blood chemistry, biomarkers, and hemodynamic status were monitored throughout the study.

Shifts in hemodynamic status were observed in the test group relative to the control group indicating improved cardiac function and decreased sympathetic nervous system tone. For example, heart rate and mean arterial pressure decreased, while contractility increased relative to controls. These comparative outcomes were supported by positive shifts in biomarkers such as pro-BNP and NGAL, relative to controls. As an example, animals with implant produced about 35% more urine with about 21% higher creatinine content, resulting in about 52% less increase in serum creatinine as a result of the diuretic challenge. Table 1 shows the results for the study.

**Table 1: Study Results**

| | pre-implant | | 35 days post implant | | Change during study | |
|---|---|---|---|---|---|---|
| | test | control | test | control | test | control |
| Mean Rate (bpm) | 85 | 86 | 80 | 90 | -6 | 4 |
| Mean Arterial Pressure (mmHg) | 87 | 68 | 83 | 85 | -9 | -2 |
| Mean Heart Contractility (mmHg/s) | 1384 | 1367 | 1581 | 1299 | 197 | -68 |
| Mean Plasma NGAL Biomarker (ng/ml) | 1669 | 1234 | 1502 | 1442 | -167 | 208 |
| Mean nt-proBNP Biomarker (pg/ml) | 5.5 | 6.22 | 6.14 | 8.62 | 0.64 | 2.4 |

At the end of the study, all animals (test and control) received an 80 mg bolus of Lasix (a diuretic). In this model healthy animals experience minimal change in serum creatinine levels while animals in heart failure experience a significant increase. This test was conducted to illustrate the impact of the device when the kidney is stressed. Table 2 shows the post-bolus results.

**Table 2: Post-Bolus Results**

| | pre-diuretic | | 4 hours post-diuretic | | Change during study | |
|---|---|---|---|---|---|---|
| | test | control | test | control | test | control |
| Mean Serum Creatinine | 0.9 | 0.77 | 1.05 | 1.08 | 0.15 | 0.31 |
| Mean Urine Creatinine (mg/dL) | | | 46 | 38 | | |
| Mean Urine Output (ml) | | | 348 | 258 | | |

The above results show that a flow restricting device, as discussed in detail above, placed in the aorta distal to the renal arteries may help decrease the symptoms of fluid overload and cardiac stress associated with heart failure. The devices are shown to increase blood pressure proximal to the stenosis and, in doing so, increase kidney perfusion pressure, thus increasing kidney perfusion. A secondary effect of this device is the reduction in the activation of the RAAS system. Effectiveness of the device was based on assessment of central hemodynamics, left ventricular (LV) function and renal function.

Previous studies have found that induced stenosis has little effect on flow or pressure until it reaches about 40%, after which the impact is dependent on artery diameter and blood flow rate. Based on the above animal study, however, it has been discovered that there is a threshold above which the impact dramatically increases. The regime for stenosis, based on these results, is between 40%-80%, and more particularly between about 50% - 70%. Clinically, measurement of ankle pressure, Doppler ultrasound velocity, or other hemodynamic parameters in the lower limbs can be employed to optimize the magnitude of the induced stenosis while ensuring adequate limb perfusion.

Examples of synthetic polymers (which may be used as a graft component) include, but are not limited to, nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, **polyamides,** their mixtures, blends and copolymers are suitable as a graft material. In one embodiment, said graft is made from a class of polyesters such as polyethylene terephthalate including DACRON^{®} and MYLAR ^{®} and polyaramids such as KEVLAR^{®}, polyfluorocarbons such as polytetrafluoroethylene (PTFE) with and without copolymerized hexafluoropropylene (TEFLON^{®}. or GORE-TEX^{®}.), and porous or nonporous polyurethanes. In certain instances, the graft comprises expanded fluorocarbon polymers (especially PTFE) materials described in British. Pat. No. 1,355,373; 1,506,432; or 1,506,432 or in U.S. Pat. No. 3,953,566; 4,187,390; or 5,276,276. Included in the class of preferred fluoropolymers are polytetrafluoroethylene (PTFE), fluorinated ethylene propylene (FEP), copolymers of tetrafluoroethylene (TFE) and perfluoro(propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is ePTFE. In certain instances, the graft comprises a combination of said materials listed above. In certain instances, the graft is substantially impermeable to bodily fluids. Said substantially impermeable graft can be made from materials that are substantially impermeable to bodily fluids or can be constructed from permeable materials treated or manufactured to be substantially impermeable to bodily fluids (e.g. by layering different types of materials described above or known in the art).

Additional examples of graft materials include, but are not limited to, vinylidinefluoride/hexafluoropropylene hexafluoropropylene (HFP), tetrafluoroethylene (TFE), vinylidenefluoride, 1-hydropentafluoropropylene, perfluoro(methyl vinyl ether), chlorotrifluoroethylene (CTFE), pentafluoropropene, trifluoroethylene, hexafluoroacetone, hexafluoroisobutylene, fluorinated poly(ethylene-co-propylene (FPEP), poly(hexafluoropropene) (PHFP), poly(chlorotrifluoroethylene) (PCTFE), poly(vinylidene fluoride (PVDF), poly(vinylidene fluoride-co-tetrafluoroethylene) (PVDF-TFE), poly(vinylidene fluoride-co-hexafluoropropene) (PVDF-HFP), poly(tetrafluoroethylene-co-hexafluoropropene) (PTFE-HFP), poly(tetrafluoroethylene-co-vinyl alcohol) (PTFE-VAL), poly(tetrafluoroethylene-co-vinyl acetate) (PTFE-VAC), poly(tetrafluoroethylene-co-propene) (PTFEP) poly(hexafluoropropene-co-vinyl alcohol) (PHFP-VAL), poly(ethylene-co-tetrafluoroethylene) (PETFE), poly(ethylene-co-hexafluoropropene) (PEHFP), poly(vinylidene fluoride-co-chlorotrifluoroe-thylene) (PVDF-CTFE), and combinations thereof, and additional polymers and copolymers described in U.S. Publication 2004/0063805. Additional polyfluorocopolymers include tetrafluoroethylene (TFE)/perfluoroalkylvinylether (PAVE). PAVE can be perfluoromethylvinylether (PMVE), perfluoroethylvinylether (PEVE), or perfluoropropylvinylether (PPVE), as essentially described in U.S. Publication 2006/0198866 and U.S. Pat. No. 7,049,380. Other polymers and copolymers include, polylactide, polycaprolacton-glycolide, polyorthoesters, polyanhydrides; poly-aminoacids; polysaccharides; polyphosphazenes; poly(ether-ester) copolymers, e.g., PEO-PLLA, or blends thereof, polydimethyl-siolxane; poly(ethylene-vingylacetate); acrylate based polymers or copolymers, e.g., poly(hydroxyethyl methylmethacrylate, polyvinyl pyrrolidinone; fluorinated polymers such as polytetrafluoroethylene; cellulose esters and any polymer and copolymers described in U.S. Publication 2004/0063805.

The graft components, as discussed herein, may be attached to the self-expanding stent elements by using a coupling member that is generally a flat ribbon or tape having at least one generally flat surface. In certain instances, the tape member is made from expanded PTFE (ePTFE) coated with an adhesive. The adhesive may be a thermoplastic adhesive. In certain instances, the thermoplastic adhesive may be fluorinated ethylene propylene (FEP). More specifically, an FEP-coated side of the ePTFE may face toward and contacts an exterior surface of the self-expanding stent and graft component, thus attaching the self-expanding stent to the graft component. Materials and method of attaching a stent to the graft is discussed in U.S. Pat. No. 6,042,602 to Martin.

The stent elements discussed herein can be fabricated from a variety of biocompatible materials. These materials may include 316L stainless steel, cobalt-chromium-nickel-molybdenum-iron alloy ("cobalt-chromium"), other cobalt alloys such as L605, tantalum, nickel-titanium alloys (e.g., Nitinol), or other biocompatible metals. In certain instances, as discussed in detail above, the stent (and graft) may be self-expanding. The prosthesis may be balloon expandable

A variety of materials variously metallic, super elastic alloys, such as Nitinol, are suitable for use in these stents. Primary requirements of the materials are that they be suitably springy even when fashioned into very thin sheets or small diameter wires. Various stainless steels which have been physically, chemically, and otherwise treated to produce high springiness are suitable as are other metal alloys such as cobalt chrome alloys (e.g., ELGILOYO), platinum/tungsten alloys, and especially the nickel-titanium alloys (e.g., Nitinol).

## Claims

1. An apparatus comprising:
an implantable flow restriction device ( 800) including a first portion (808), a second portion (810), and a channel (812) therebetween, wherein the first portion and the second portion are diametrically adjustable to alter the blood flow through the channel (812), wherein the implantable flow restriction device (800) is configured to implant within an aorta of a patient; and wherein the first portion (808) and the second portion (810) are configured to dimensionally adjust the implantable flow restriction device ( 800) to induce stenosis of the aorta distal of the at least one branch vessel of between 40% and 80% and alter blood flow into the at least one branch vessel of the aorta while maintaining a substantially unrestricted blood flow within the aorta proximal to the at least one branch vessel.

2. The apparatus of claim 1, wherein the implantable flow restriction device (800) is configured to induce stenosis of the aorta distal of the at least one branch vessel of the aorta between 50% and 70%.

3. The apparatus of any one of claims 1-2, wherein the implantable flow restriction device (800) is configured to alter pressure within the aorta to alter blood flow into the at least one branch vessel of the aorta.

4. The apparatus of any one of claims 1-3, wherein the implantable flow restriction device (800) is configured to increase pressure distal to the at least one branch vessel of the aorta to increase blood flow into the at least one branch vessel of the aorta.

## Patentansprüche

1. Vorrichtung, umfassend:
eine implantierbare Flussbegrenzungsvorrichtung (800), die einen ersten Abschnitt (808), einen zweiten Abschnitt (810) und einen Kanal (812) dazwischen enthält, wobei der erste Abschnitt und der zweite Abschnitt diametral einstellbar sind, um den Blutfluss durch den Kanal (812) zu verändern, wobei die implantierbare Flussbegrenzungsvorrichtung (800) zum Implantieren innerhalb einer Aorta eines Patienten konfiguriert ist; und wobei der erste Abschnitt (808) und der zweite Abschnitt (810) konfiguriert sind, um die implantierbare Flussbegrenzungsvorrichtung (800) dimensional anzupassen, um eine Stenose der Aorta distal von dem mindestens einen Zweiggefäß von zwischen 40 % und 80 % zu induzieren und den Blutfluss in das mindestens eine Zweiggefäß der Aorta zu verändern, während ein im Wesentlichen uneingeschränkter Blutfluss innerhalb der Aorta proximal zu dem mindestens einen Zweiggefäß aufrechterhalten wird.

2. Vorrichtung nach Anspruch 1, wobei die implantierbare Flussbegrenzungsvorrichtung (800) konfiguriert ist, um eine Stenose der Aorta distal von dem mindestens einen Zweiggefäß der Aorta zwischen 50 % und 70 % zu induzieren.

3. Vorrichtung nach einem der Ansprüche 1 bis 2, wobei die implantierbare Flussbegrenzungsvorrichtung (800) konfiguriert ist, um den Druck innerhalb der Aorta zu verändern, um den Blutfluss in das mindestens eine Zweiggefäß der Aorta zu verändern.

4. Vorrichtung nach einem der Ansprüche 1 bis 3, wobei die implantierbare Flussbegrenzungsvorrichtung (800) konfiguriert ist, um den Druck distal zu dem mindestens einen Zweiggefäß der Aorta zu erhöhen, um den Blutfluss in das mindestens eine Zweiggefäß der Aorta zu erhöhen.

## Revendications

1. Appareil comprenant :
un dispositif de restriction de flux implantable (800) comprenant une première partie (808), une seconde partie (810) et un canal (812) entre elles, ladite première partie et ladite seconde partie étant diamétralement réglables pour modifier le flux sanguin à travers le canal (812), ledit dispositif de restriction de flux implantable (800) étant conçu pour s'implanter dans l'aorte d'un patient ; et
ladite première partie (808) et ladite seconde partie (810) étant conçues pour ajuster dimensionnellement le dispositif de restriction de flux implantable (800) pour induire une sténose de l'aorte distale dudit au moins un vaisseau ramifié d'entre 40 % et 80 % et modifier le flux sanguin dans ledit au moins un vaisseau ramifié de l'aorte tout en maintenant un flux sanguin sensiblement non restreint à l'intérieur de l'aorte à proximité dudit au moins un vaisseau ramifié.

2. Appareil selon la revendication 1, ledit dispositif de restriction de flux implantable (800) étant conçu pour induire une sténose de l'aorte distale dudit au moins un vaisseau ramifié de l'aorte comprise entre 50 % et 70 %.

3. Appareil selon l'une quelconque des revendications 1 à 2, ledit dispositif de restriction de flux implantable (800) étant conçu pour modifier la pression à l'intérieur de l'aorte afin de modifier le flux sanguin dans ledit au moins un vaisseau ramifié de l'aorte.

4. Appareil selon l'une quelconque des revendications 1 à 3, ledit dispositif de restriction de flux implantable (800) étant conçu pour augmenter la pression distale sur ledit au moins un vaisseau ramifié de l'aorte pour augmenter le flux sanguin dans ledit au moins un vaisseau ramifié de l'aorte.
